# EUROPEAN PATENT SPECIFICATION

(11) **EP 4 324 511 B1**
(45) Date of publication and mention of the grant of the patent: **17.12.2025**
(21) Application number: 22787354.4
(22) Date of filing: 25.03.2022
(51) Int. Cl.: A61N 1/36, A61N 1/372, A61N 1/378

(54) **IMPLANTABLE NERVE STIMULATOR SYSTEM**
IMPLANTIERBARES NERVENSTIMULATIONSSYSTEM
SYSTÈME DE STIMULATEUR NERVEUX IMPLANTABLE

(30) Priority: 16.04.2021 CN 202110412753; 16.04.2021 CN 202110412431
(43) Date of publication of application: 21.02.2024
(73) Proprietor: BEIJING LEADING INNOVATION MEDICAL VALLEY CO., LTD, Beijing 100162 (CN)
(72) Inventor: XU, Tianrui, Beijing 100162 (CN)
(74) Representative: EP&C
(86) International application number: PCT/CN2022/082960
(87) International publication number: WO 2022/218125

(56) References cited:
- CN-A- 101 244 312
- CN-A- 101 244 312
- CN-A- 101 773 701
- CN-A- 104 096 313
- CN-A- 112 972 896
- CN-A- 113 274 640
- CN-U- 215 084 264
- US-A1- 2013 238 048
- US-A1- 2018 169 423
- US-A1- 2020 222 703

## Description

### Technical Field

The present disclosure relates to an implantable neural stimulation system comprising an extracorporeal energy controller and a corresponding implantable neurostimulator. The present disclosure further relates to the extracorporeal energy controller.

### Background Art

A neural stimulation system comprising an implantable neurostimulator has been widely used in the medical field. In this system, the implantable neurostimulator is implanted into the patient's body to achieve treatment of the patient site.

A traditional implantable neurostimulator requires a built-in battery for power supply. After the battery runs out of charge, it is necessary to take the neurostimulator out off the patient's body in order to reinstall the battery. In addition, when doctors need to change a treatment regimen, it is also necessary to take the neurostimulator out off the patient's body in order to reconfigure the treatment regimen. The treatment regimen includes, for example, pulse width, pulse frequency, and the like of a stimulation pulse. This is undoubtedly painful for a patient having a long treatment time.

To address this pain, a radio-frequency controlled neural stimulation system has emerged, and such nerve stimulator system has been disclosed in patent application publication US 2013/238048 A1 and Chinese invention patents CN104080509B and CN107789730B, wherein the implantable neurostimulator conducts radio frequency communication and radio frequency energy transmission with an extracorporeal energy controller which provides an electrical stimulation pulse in real-time to drive a stimulation electrode of the implantable neurostimulator, thereby applying a stimulation signal to the patient's treatment site; and provides radio frequency energy to the implantable neurostimulator so as to maintain operation of the implantable neurostimulator.

Compared to previous implantable neural stimulation systems, the radio-frequency based neurostimulator can obtain almost endless power supply, so there is no need to worry about battery depletion. Moreover, this radio-frequency based implantable neurostimulator can adjust the electrical stimulation pulse at any time by the extracorporeal energy controller according to the treatment regimen. Therefore, there is no need to worry about repeated implantation issues caused by battery depletion and changes in treatment regimen.

However, there are still many shortcomings in the radio-frequency based neural stimulation system of the prior art.

Due to the fact that the extracorporeal energy controller needs to provide electrical energy and input signals (such as various stimulation pulse sequences) simultaneously to the implantable neurostimulator, and real-time monitoring of the working status of the implantable neurostimulator is required, it may not be possible to achieve real-time operation of the implantable neurostimulator, which also has adverse effects on the treatment process. To address this issue, CN107789730B adopts a dual frequency operating mode, which increases the complexity and manufacturing cost of the product and may lead to an increase in size of the implantable neurostimulator. And this increase in size is obviously not conducive to the implantation of neurostimulators.

In addition, since the electrical stimulation pulse of the implantable neurostimulator is provided in real-time by the extracorporeal energy controller, it is necessary to ensure reliable communication between the neurostimulator implanted in the patient's body and the extracorporeal energy controller. And the reliability of this communication will be influenced by many factors. For example, when the extracorporeal energy controller is away from the patient due to some factors, or when the extracorporeal energy controller is accidentally impacted or damaged, even for a very short period of time, it will have adverse effects on the treatment process of the implantable neurostimulator.

The information disclosed in the background part of the present disclosure is only intended to increase an understanding of the overall background of the present disclosure and should not be regarded as acknowledging or implying in any form that the information constitutes the prior art that is already well-known to those skilled in the art.

### Summary of the Invention

A purpose of the present disclosure is to provide an extracorporeal energy controller of a neural stimulation system, which forms a neural stimulation system with an implantable neurostimulator through radio frequency communication. The neural stimulation system may also comprise an upper computer software for easy operation and setting, with its characteristics being that the operation control of the implantable neurostimulator is not completed by the extracorporeal energy controller, but by a main control chip built-in the implantable neurostimulator; the extracorporeal energy controller is used to configure clinical treatment parameters of the implantable neurostimulator and store operation data from the implantable neurostimulator, and adjust the transmission power in response to instructions of the implantable neurostimulator. This overcomes issues of treatment safety and product complexity caused by the need for real-time communication in the implantable nerve stimulator system of the prior art.

The present invention provides an implantable nerve stimulator system, comprising an implantable neurostimulator having a stimulator antenna module and an extracorporeal energy controller having an energy controller antenna module, through the stimulator antenna module and the energy controller antenna module, the implantable neurostimulator communicating with and receiving electrical energy from the extracorporeal energy controller through radio frequency, wherein the implantable neurostimulator has a main control CPU, a main control memory for storing control information containing clinical stimulation parameters, and a stimulation electrodes, the main control CPU using the clinical stimulation parameters to actively generate stimulation pulse sequences and applying the stimulation pulse sequences to the stimulation electrodes.

According to the implantable nerve stimulator system of the present invention, the implantable neurostimulator further comprises a rectification energy storage circuit for storing the received electrical energy and a front measurement feedback circuit for measuring the electrical energy storage amount in the rectification energy storage circuit, and when the electrical energy storage amount is insufficient, the main control CPU sends a power adjustment instruction to the extracorporeal energy controller which adjusts transmission power of the extracorporeal energy controller based on the received power adjustment instruction.

According to the implantable nerve stimulator system of the present invention, preferably the implantable neurostimulator further comprises a post measurement feedback circuit for measuring real-time stimulation parameters on the stimulation electrode, which are transmit to the main control CPU which stores the real-time stimulation parameters in the main control memory, and periodically sends the real-time stimulation parameters to the extracorporeal energy controller, or sends the real-time stimulation parameters to the extracorporeal energy controller in response to a data read instruction; the extracorporeal energy controller comprises a storage unit to store the real-time stimulation parameters received from the implantable neurostimulator.

According to the implantable nerve stimulator system of the present invention, the extracorporeal energy controller further comprises an input device, a display device, and a power supply; the input device and the display device are used to achieve human-machine interaction, so as to send the control information to the implantable neurostimulator; the control information comprises an instruction for modifying the clinical stimulation parameters.

According to the implantable nerve stimulator system of the present invention, preferably- the control information further comprises an up/down shift instruction to adjust stimulation intensity of the stimulation pulse sequences as needed.

According to the implantable nerve stimulator system of the present invention, preferably the extracorporeal energy controller further comprises a storage unit that stores operating program of the extracorporeal energy controller, information coming from the input device, and data received from the implantable neurostimulator;
The control information also comprises the data read instruction to read operation data comprising real-time stimulation parameters from the implantable neurostimulator at any time.

According to the implantable nerve stimulator system of the present invention, preferably the implantable nerve stimulator system further comprises an upper computer serving as a control and information processing station, and the extracorporeal energy controller further comprises an upper computer communication module, through which the upper computer communicates with the extracorporeal energy controller, thereby sending an instruction to the implantable neurostimulator through the extracorporeal energy controller, or reads data from the extracorporeal energy controller.

According to the implantable nerve stimulator system of the present invention, preferably the upper computer communication module is a wireless communication module such as Bluetooth.

According to the implantable nerve stimulator system of the present invention, preferably the upper computer has an upper computer software, through which a user sends an instruction to the extracorporeal controller of a neural stimulation system to operate the extracorporeal controller of the neural stimulation system, or operate the implantable neurostimulator through the extracorporeal controller of the neural stimulation system, the operations comprising setting, measuring, programming and data management of the clinical stimulation parameters, the data involved in the data management comprising measurable relevant parameters generated during operation of the extracorporeal controller and the implantable neurostimulator of the neural stimulation system, modified data and observation variables.

According to the implantable nerve stimulator system of the present invention, preferably the upper computer software is capable of operating the upper computer to connect to a network or an internal server for backup and update of the program and data.

The implantable nerve stimulator system of the present disclosure can achieve the following beneficial technical effects:
Due to the implementation of electrical pulse stimulations based on the combination of treatment parameters stored in the main control memory of the implantable neurostimulator, RF electrical energy needs to be provided only by the extracorporeal energy controller, without the need to obtain real-time stimulation signals containing stimulation electrical pulses from the extracorporeal energy controller. Therefore, the operation reliability of the implantable neurostimulator is improved, and there is no need to worry about treatment failure caused by sudden interruption of communication or poor communication.

Due to the energy storage circuit self-equipped in the implantable neurostimulator, it can ensure a short period of electrical energy supply in the event of sudden interruption of communication or poor communication, without interrupting treatment.

Due to the fact that the memory of the implantable neurostimulator can store various operating parameters and send these data to the extracorporeal energy controller during treatment intervals or when communication is not busy, it can further ensure smooth communication when communication is needed, thereby improving the performance of the device.

The methods and devices of the present disclosure have other characteristics and advantages that will be apparent from the accompanying drawings and subsequent specific embodiments incorporated herein, or will be described in detail in the accompanying drawings and subsequent specific embodiments, which together serve to explain the specific principles of the present disclosure.

### Brief Description of Drawings

Figure 1 shows a schematic block diagram of an embodiment of a neural stimulation system comprising an extracorporeal energy controller according to the present disclosure.
Figure 2 shows a schematic block diagram of another embodiment of a neural stimulation system comprising an extracorporeal energy controller according to the present disclosure.
Figure 3 shows a schematic block diagram of an extracorporeal energy controller of a neural stimulation system according to the present disclosure.
Figure 4 shows a schematic block diagram of an implantable neurostimulator of an implantable nerve stimulator system according to the present disclosure.
Figure 5 shows a schematic block diagram of another implantable neurostimulator of an implantable nerve stimulator system according to the present disclosure.

It should be understood that the accompanying drawings do not necessarily have to be drawn to scale, as they illustrate various features of the basic principles of the present disclosure that have been simplified to some extent.

The specific design features of the present disclosure disclosed herein, including specific dimensions, orientation, positioning, and shape, will be partially determined by the application and usage environment for the specific purpose.

In these drawings, in figures running through the drawings, the reference numerals refer to same or equivalent parts of the present disclosure.

### Detailed Description of Embodiments

Now, specific reference will be made to the various embodiments of the present disclosure, and examples of these embodiments are shown in the accompanying drawings and the following description.

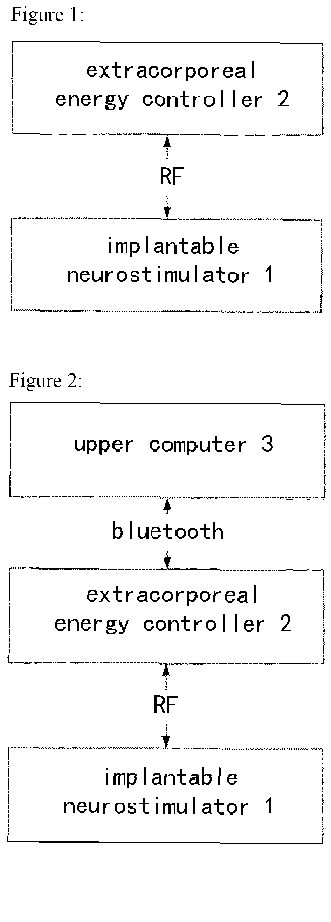

Figure 1 shows a schematic block diagram of an embodiment of a neural stimulation system comprising an extracorporeal energy controller according to the present disclosure. As shown in Figure 1, the neural stimulation system includes two parts: an implantable neurostimulator 1 and an extracorporeal energy controller 2.

Figure 2 shows a schematic block diagram of another embodiment of a neural stimulation system comprising an extracorporeal energy controller according to the present disclosure. Compared to the embodiment in Figure 1, the embodiment in Figure 2 is added with an upper computer 3. The upper computer is not necessary. Adding an upper computer can help improve human-computer interaction functions, making it easier for doctors or patients to operate the neural stimulation system and convenient to provide more complex functions for the neural stimulation system.

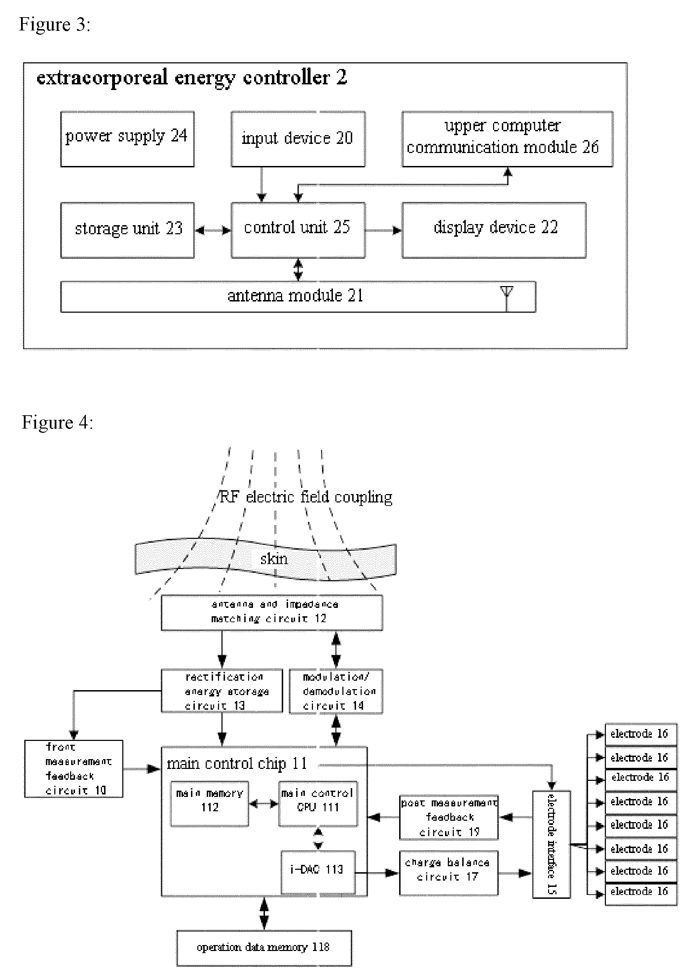

Figure 3 shows a schematic block diagram of an extracorporeal energy controller of a neural stimulation system according to the present disclosure.

As shown in Figures 1 and 2, the extracorporeal energy controller 2 of the neural stimulation system of the present disclosure transmits electrical energy to and communicates with the implantable neurostimulator 1 through radio frequency. As shown in Figure 3, the extracorporeal energy controller 2 of the implantable neural stimulation system of the present disclosure comprises: an input device 20 through which the extracorporeal energy controller 2 receives information; an antenna module 21 that is in radio-frequency coupling with a stimulator antenna of the implantable neurostimulator 1 to send input signals containing electrical energy and control information to the implantable neurostimulator 1, and can receive instructions and data from the implantable neurostimulator 1; a display device 22 for displaying current state of the extracorporeal energy controller 2 and information coming from input device 20, as well as data and instructions received from implantable neurostimulator 1; a storage unit 23 storing running programs of the extracorporeal energy controller 2, information coming from the input device 20, and data received from the implantable neurostimulator 1; a power supply 24 for supplying power to the extracorporeal energy controller of the entire neural stimulation system; and a control unit 25 respectively connected to the input device 20, the antenna module 21, the power supply 24 and the display device 22 to control operation of the entire extracorporeal energy controller 2.

A user can operate the input device. The information coming from the input device 20 can include information for configuring the extracorporeal energy controller 2, information for configuring the implantable neurostimulator 1, and instructions to read data from the implantable neurostimulator 1. In addition, by operating the input device 20, any information of the extracorporeal energy controller 2 itself, including information stored in the storage unit 23 (such as operation data coming from the implantable neurostimulator 1), can also be displayed.

The input device 20 can be any device suitable for inputting information, such as buttons, handwriting screens, voice input microphones, etc.

Preferably, the input device 20 has a stimulation intensity adjustment unit that can adjust the stimulation intensity of the implantable neurostimulator 1 in an up/down shifting manner. The stimulation intensity adjustment unit can be an up/down shifting button, or a virtual button displayed on the screen.

The display device 22 can be any device capable of displaying information, such as a liquid crystal display screen, LED display, etc.

The storage unit 23 is preferably a non-volatile memory, so that data can be stored even after a power outage. In this way, the extracorporeal energy controller 2 of the neural stimulation system needs to be configured according to the treatment regimen for each patient only before the start of each treatment phase, which can be applied to the entire treatment phase. Therefore, it avoids the need for doctors to frequently set the extracorporeal energy controller 2 of the neural stimulation system.

The power supply 24 can be an ordinary battery or a rechargeable battery.

The above instruction received from the implantable neurostimulator 1 is an instruction to adjust the transmission power of the extracorporeal energy controller, and according to this instruction, the control unit 25 adjusts the transmission power of the antenna module 21 to meet the operational requirements of the implantable neurostimulator 1. This way of adjusting the transmission power based on the operational requirements of the implantable neurostimulator 1 is clearly more helpful in ensuring the reliable operation of the implantable neurostimulator 1.

The extracorporeal energy controller 2 of the neural stimulation system of the present disclosure may also have an upper computer communication module 26 that receives instructions from the upper computer 3 and sends data to the upper computer 3. The instructions are used for configuring the extracorporeal energy controller 2 or the implantable neurostimulator 2 of the neural stimulation system; alternatively, they are used to transmit various data from the extracorporeal energy controller 2 of the neural stimulation system and data from the implantable neurostimulator 1 to the upper computer 3.

The upper computer 3 may have a dedicated upper computer software, through which a user sends an instruction to the extracorporeal energy controller 2 of the neural stimulation system to operate the extracorporeal energy controller 2 of the neural stimulation system, or operate the implantable neurostimulator 1 through the extracorporeal energy controller 2 of the neural stimulation system, the operations including setting, measuring, programming and data management of the clinical stimulation parameters.

The upper computer software may also operate the upper computer 3 to connect to a network or an internal server for backup and update of the program and data.

Obviously, the upper computer 3 and the included upper computer software may improve the convenience of operation and help to set up more complex treatment regimen.

The upper computer communication module 26 of the extracorporeal energy controller 2 of the neural stimulation system of the present disclosure can be a wireless communication module, thereby exchanging instruction and data with the upper computer 3 in wireless communication. Obviously, the wireless communication make the connection between the extracorporeal energy controller 2 of the neural stimulation system and the upper computer 3 more convenient, thereby improving the convenience of operation and the simplicity of product design.

The wireless communication module mentioned above can be a Bluetooth module.

The extracorporeal energy controller 2 of the neural stimulation system of the present disclosure is often designed to be worn in a portable form, so as to be able to move to any place with the patient. This portable design needs to minimize the size of the product, so the battery size will also be minimized, which requires full consideration of the energy-saving design of the device. Bluetooth communication has the characteristic of low power consumption, which can well meet this energy-saving requirement.

The implantable neurostimulator 1, as an in vivo therapeutic device, requires high safety. That is, to prevent illegal operators or illegal extracorporeal control devices from interfering with the implantable neurostimulator 1. Bluetooth also provides two password protections, which can effectively prevent this risk of illegal intrusion.

Optionally, the wireless communication module mentioned above may also be a WIFI module. Depending on the difference of treatment regimens, some treatment regimen may generate a large amount of data, which increases the amount of data that needs to be transmitted. WIFI communication has fast transmission rate, which helps to meet this demand.

The extracorporeal energy controller 2 of the neural stimulation system of the present disclosure does not need to send real-time stimulation signals containing stimulation electrical pulses to the implantable neurostimulator 1, but only needs to provide radio-frequency electrical energy. Therefore, even if communication is interrupted or blocked due to unexpected events, treatment failure will not occur.

Due to the fact that the implantable neurostimulator 1 can send these data to the extracorporeal energy controller during treatment intervals or when communication is not busy, the extracorporeal energy controller 2 of the neural stimulation system of the present disclosure can further ensure smooth communication when communication is needed, thereby improving the performance of device. For example, when a doctor or patient operates the extracorporeal controller to send instructions to the implantable neurostimulator, the implantable neurostimulator will not send data outward to ensure smooth communication.

The extracorporeal energy controller 2 of the neural stimulation system of the present disclosure can provide stable power supply to the implantable neurostimulator 1 by receiving an instruction for adjusting the transmission power from the implantable neurostimulator and responding to the instruction to adjust the transmission power of the antenna module.

As shown in Figure 1, the implantable nerve stimulator system of the present disclosure comprises an implantable neurostimulator 1 and an extracorporeal energy controller 2, the implantable neurostimulator 1 communicating with and receiving electrical energy from the extracorporeal energy controller 2 through radio frequency, wherein the implantable neurostimulator has a main control CPU, a main control memory for storing control information containing clinical stimulation parameters, and a stimulation electrodes, the main control CPU utilizing the clinical stimulation parameters to actively generate stimulation pulse sequences and applying the stimulation pulse sequences to the stimulation electrodes.

In the above implantable nerve stimulator system, the implantable neurostimulator 1 further comprises a rectification energy storage circuit for storing the received electrical energy and a front measurement feedback circuit for measuring the electrical energy storage amount in the rectification energy storage circuit; and when the electrical energy storage amount is insufficient, the main control CPU sends a power adjustment instruction to the extracorporeal energy controller which adjusts transmission power of the extracorporeal energy controller based on the received power adjustment instruction.

In the above implantable nerve stimulator system, the implantable neurostimulator 1 further comprises a post measurement feedback circuit for measuring real-time stimulation parameters on the stimulation electrode, which are transmit to the main control CPU which stores the real-time stimulation parameters in the main control memory, and periodically sends the real-time stimulation parameters to the extracorporeal energy controller, or sends the real-time stimulation parameters to the extracorporeal energy controller in response to a data read instruction; the extracorporeal energy controller comprises a storage unit to store the real-time stimulation parameters received from the implantable neurostimulator.

In the above implantable nerve stimulator system, the extracorporeal energy controller 2 further comprises an input device, a display device, and a power supply; the input device and the display device are used to achieve human-machine interaction, so as to send the control information to the implantable neurostimulator; the control information includes an instruction for temporarily modifying clinical stimulation parameters, and the temporarily modified clinical stimulations parameters will be restored to the original set values after the system is restarted.

In the above implantable nerve stimulator system, the control information further includes an up/down shift instruction to adjust stimulation intensity of the stimulation pulse sequences as needed.

In the above implantable nerve stimulator system, the control information also includes the data read instruction to read operation data including real-time stimulation parameters from the implantable neurostimulator at any time.

Figure 2 shows a schematic block diagram of another embodiment comprising an implantable nerve stimulator system of the present disclosure.

Compared to the embodiment in Figure 1, the embodiment in Figure 2 is added with an upper computer 3. The upper computer is not necessary. Adding an upper computer helps to improve human-computer interaction functions, making it easier for doctors or patients to operate the neural stimulation system and convenient to provide more complex functions for the neural stimulation system.

In the implantable nerve stimulator system of the present disclosure as shown in Figure 2, an upper computer serving as a control and information processing station is added, and the extracorporeal energy controller further comprises an upper computer communication module, through which the upper computer communicates with the extracorporeal energy controller, thereby sending an instruction to the implantable neurostimulator through the extracorporeal energy controller, or reads data from the extracorporeal energy controller.

The above upper computer communication module may be a wireless communication module, such as a Bluetooth communication module, to make communication connections more convenient. The above upper computer may also have a data analysis and management system, which is used to analyze and manage the data read from the extracorporeal energy controller, thereby helping to specify and modify combination of clinical stimulation parameters.

Figure 4 shows a schematic block diagram of an implantable neurostimulator of a neural stimulation system of the present disclosure As shown in Figure 4, the implantable neurostimulator 1 of the present disclosure that communicates with and receives electrical energy from an extracorporeal energy controller through radio frequency, comprises: a main control chip 11 that includes a main CPU 111, a main control memory 112, and a digital to analog conversion current source circuit (i-DAC) 113; a stimulator antenna and its impedance matching circuit 12, which are radio-frequency coupled with the extracorporeal energy controller to receive input signals containing electrical energy and control information from the extracorporeal energy controller, and are capable of sending data to the extracorporeal energy controller; a rectification energy storage circuit 13 connected to the impedance matching circuit 12 and the main control chip 11, respectively, so as to extract electrical energy from the received input signal and store electrical energy, and supply power to the main control chip 11; a modulation/demodulation circuit 14 connected to the impedance matching circuit 12 and the main control chip 11 to extract control information from the received input signal and transmit the control information to the main control chip 11, and modulates data sent by the main control chip 11, which is then transmitted to the impedance matching circuit, and sent to the extracorporeal energy controller through the stimulator antenna; an electrode interface 15 connected to the main control chip 11 and receives polarity assignment information from the main control chip 11, and receives stimulation pulse sequences from the digital to analog conversion current source circuit 113; one or more stimulation electrode(s) 16 connected to the electrode interface 15 which assigns the stimulation pulse sequences to each corresponding stimulation electrode 16 based on the polarity assignment information; wherein the main control memory 112 is stored with a control program and the received control information, the main control CPU runs the control program to control the digital to analog conversion current source circuit 113 to generate the stimulation pulse sequences based on the control information, and the control information includes a combination of clinical stimulation parameters which is a combination of parameters consisting of polarity assignment information parameters, pulse width parameters, pulse amplitude parameters and pulse frequency parameters.

The main control memory 112 is preferably a non-volatile memory, so that data can be stored even after a power outage. In this way, the implantable neurostimulator 1 needs to be configured according to the treatment regimen for each patient only before the start of each treatment phase, which can be applied to the entire treatment phase. Therefore, it avoids the need for doctors to frequently set the implantable neurostimulator 1.

The combination of clinical stimulation parameters may comprise multiple groups, each group of which having its own code, and the control information further includes codes of clinical stimulation parameters which correspond one-to-one with the codes of multiple groups of the combination of clinical stimulation parameters stored in the main control memory. In this way, a user (doctor or patient) can directly retrieve a corresponding treatment regimen (corresponding to the corresponding clinical stimulation parameter combination) by operating the extracorporeal energy controller according to the treatment process, which avoids the hassle of frequently configuring the implantable neurostimulator as the patient's condition improves.

The above control information further includes an up/down shift control instruction, in response to which the main control chip 11 adjusts pulse intensity of the stimulation pulse sequence in a step-by-step manner. In this way, the patient can adjust the intensity of stimulation at any time based on its own feelings. In the implantable neurostimulator 1, the control information further includes a data read instruction, in response to which the main control CPU sends corresponding data stored in the main control memory to the extracorporeal energy controller 2. In this way, the patient or doctor can obtain combination of various treatment parameters stored in implantable neurostimulator 1, as well as the data generated by operation of the implantable neurostimulator 1.

The implantable neurostimulator uses stimulation pulse sequences to treat patients. When the pulse frequency is high, the charge between adjacent stimulation pulses cannot be fully released, which makes the actual pulse waveform sequence different from the pulse waveform sequence required for the treatment. This will affect the therapeutic effect and also reduce the lifespan of the implantable neurostimulator itself.

In the implantable neurostimulator 1, the parameters of the combination of clinical stimulation parameters also include a charge balance time, the length of the charge balance time is sufficient to ensure full release of charges between adjacent electrical stimulation pulses, thereby achieving passive charge balance. This hence overcomes the problem of the existing neurostimulators where the charge between adjacent electrical stimulation pulses cannot be released.

As shown in Figure 4, in the implantable neurostimulator 1, a charge balance circuit 17 is also connected between the electrode interface 15 and the digital to analog conversion current source circuit 113 of the main control chip 11, which is capable of applying a reverse pulse to the electrode interface 15 between adjacent electrical stimulation pulses, thereby achieving active charge balance. Compared to the passive charge balance in natural discharge, the active charge balance can complete the discharge process faster. Obviously, this active charge balance allows for the use of higher stimulation pulse frequencies. But in other words, the charge balance circuit 17 is not necessary, depending on the frequency of the stimulation pulse used in implantable neurostimulator 1.

As shown in Figure 4, the implantable neurostimulator 1 also comprises an operation data memory 18 for storing various operation data generated during operation of the implantable neurostimulator. The control information received from the extracorporeal energy controller also includes a data read instruction, in response to which the main control CPU 111 sends the data stored in the operation data memory 18 to the extracorporeal energy controller 2.

It should be noted that the operation data memory 18 is not necessary, and various operation data generated during operation of the implantable neurostimulators can also be stored in a certain partition of the main control memory 112, as long as the storage capacity of the main control memory is large enough. The main control memory 18 is preferably a non-volatile memory, so that data can be stored even after a power outage. In this way, within the allowable range of storage space, the extracorporeal energy controller can retrieve the operation data of the implantable neurostimulator as needed for a period of time. It also prevents data loss due to sudden interruption of communication.

As shown in Figure 4, the implantable neurostimulator 1 further comprises a post measurement feedback circuit 19 that is respectively connected to the electrode interface 15 and the main control chip 11 to measure real-time stimulation parameters on the stimulation electrode 16, which are transmit to the main control chip 11 and stored in the operation data memory 18 by the main control chip.

The main control chip 11 can compare the real-time stimulation parameters with the clinical stimulation parameters, and corrects the stimulation signals applied to each stimulation electrode based on the comparing result.

It should be noted that the post measurement feedback circuit is not necessary. As a simplified configuration, the implantable neurostimulator may be designed to operate in a simple and reliable mode without the need to measure the working parameters of the stimulation electrode. This helps to reduce costs.

The implantable neurostimulator shown in Figure 4 further comprises a front measurement feedback circuit 10 that is provided between the rectification energy storage circuit 13 and the main control chip 11 to measure amount of real-time electrical energy storage in the rectification energy storage circuit 13 at any time, which is transmit to the main control chip 11 and stored in the operation data memory 18 by the main control chip.

The main control chip 11 evaluates whether it is necessary to adjust the radio-frequency input electrical energy based on the amount of real-time electrical energy storage. When the amount of real-time electrical energy storage is lower than a set value, the main control chip 11 sends a power adjustment instruction to an antenna of the external energy controller 2 through the stimulator antenna and its impedance matching circuit, thereby adjusting the transmission power of the extracorporeal energy controller 2.

As mentioned above, the implantable neurostimulator 1 of the present disclosure comprises a main control memory and an operation data memory. As a data management measure, the main control chip 11 can actively send data to the extracorporeal energy controller, that is, periodically send various data stored in the main control memory and/or operation data memory to the extracorporeal energy controller.

In the schematic diagram of the implantable neurostimulator shown in Figure 4, the main control chip 11 only includes a main control CPU 111, a main control memory 112, and a digital to analog conversion current source circuit (i-DAC) 113, with the other circuit part serving as the peripheral circuit.

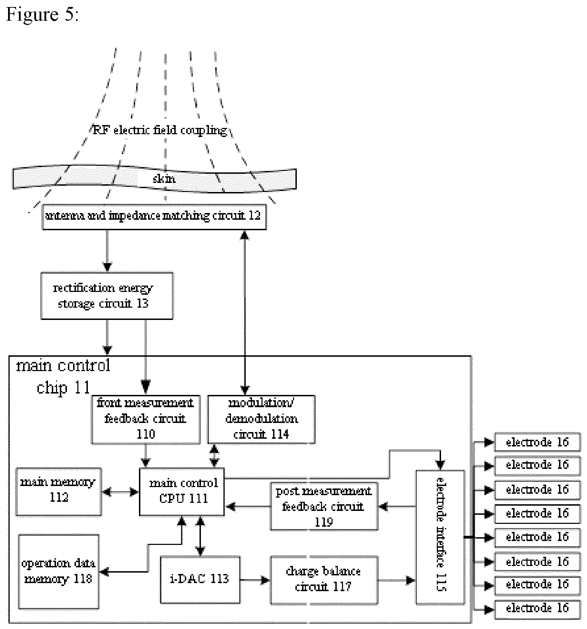

Of course, in order to balance the increase for integration to reduce volume, and the process cost, some or all of the front measurement feedback circuit, the modulation/demodulation circuit, the electrode interface, the charge balance circuit, the operation data memory, and the post measurement feedback circuit can also be designed in the main control chip 11. For example, in the schematic diagram of the implantable neurostimulator shown in Figure 5, the main control chip 11 includes the main control CPU 111, the main control memory 112, and the digital to analog conversion current source circuit (i-DAC) 113, the front measurement feedback circuit 110, the modulation/demodulation circuit 114, the electrode interface 115, the charge balance circuit 117, the operation data memory 118, and the post measurement feedback circuit 119.

In summary, the implantable neurostimulator 1 of the present disclosure is configured with parameters by an extracorporeal energy controller and is activated to operate by the extracorporeal energy controller. Once activated, the implantable neurostimulator 1 starts to operate actively with the configured parameters, completing electrode pulse stimulation treatment for the patient.

In the implantable nerve stimulator system of the present disclosure, since the implantable neurostimulator 1 is self-equipped with a rectification energy storage circuit 13, the electrical energy stored in the rectification energy storage circuit 13 supplies the entire implantable neurostimulator 1 for operation. At the same time, the rectification energy storage circuit 13 receives RF energy from the extracorporeal energy controller 2 for charging to maintain the continuous operation of the implantable neurostimulator 1. The storage amount of electrical energy in the rectification energy storage circuit 13 can be monitored by the front measurement feedback circuit. When the storage amount of electrical energy decreases, the implantable neurostimulator 1 send an instruction to the extracorporeal energy controller 2 for increasing the transmission power. Therefore, the implantable neurostimulator 1 can receive stable power supply.

In the implantable nerve stimulator system of the present disclosure, due to the implementation of electrical pulse stimulation based on the combination of treatment parameters stored in the main control memory of the implantable neurostimulator 1, RF electrical energy needs to be provided only by the extracorporeal energy controller, without the need to obtain real-time stimulation signals containing stimulation electrical pulses from the extracorporeal energy controller. Therefore, even if communication is interrupted or blocked due to unexpected events, it will not lead to treatment failure.

In the implantable nerve stimulator system of the present disclosure, since the implantable neurostimulator is self-equipped with an energy storage circuit, the RF electrical energy received from the extracorporeal energy controller can be stored, thus even if the radio frequency power supply is interrupted for a short period of time due to communication interruption or poor communication caused by an unexpected event, the implantable neurostimulator of the present disclosure can continue to operate for a period of time until communication returns to normal, thus not interrupting treatment.

In the implantable nerve stimulator system of the present disclosure, due to the fact that the memory of the implantable neurostimulator can store various operating parameters and send these data to the extracorporeal energy controller during treatment intervals or when communication is not busy, the implantable neurostimulator of the present disclosure can further ensure smooth communication when communication is needed, thereby improving the performance of the device. For example, when a doctor or patient operates an extracorporeal controller to send instructions to the implantable neurostimulator, the implantable neurostimulator will not send data outward in order to ensure smooth communication.

The aforementioned description of the specific exemplary embodiments of the present disclosure is for the purpose of illustration and exemplification. The purpose of selecting and describing exemplary embodiments is to explain the specific principles and practical applications of the present disclosure, so that other technical personnel in the art can implement and utilize various exemplary embodiments of the present disclosure, as well as various choices and changes. The scope of the present invention is intended to be limited by the accompanying claims

## Claims

1. An implantable nerve stimulator system, comprising an implantable neurostimulator (1) having a stimulator antenna module and an extracorporeal energy controller (2) having an energy controller antenna module (21), through the stimulator antenna module and the energy controller antenna module (21), the implantable neurostimulator (1) communicating with and receiving electrical energy from the extracorporeal energy controller (2) through radio frequency, wherein the implantable neurostimulator (1) has a main control CPU (111), a main control memory (112) for storing control information containing clinical stimulation parameters, and stimulation electrodes (16), and the main control CPU (111) uses the clinical stimulation parameters to actively generate stimulation pulse sequences and applies the stimulation pulse sequences to the stimulation electrodes (16);
wherein the implantable neurostimulator (1) further comprises a rectification energy storage circuit (13) for storing the received electrical energy and a front measurement feedback circuit (10) for measuring the electrical energy storage amount in the rectification energy storage circuit (13), and when the electrical energy storage amount is insufficient, the main control CPU (111) sends a power adjustment instruction to the extracorporeal energy controller (2) which adjusts transmission power based on the received power adjustment instruction;
wherein the extracorporeal energy controller (2) further comprises an input device (20), a display device (22) and a power supply (24); the input device (20) and the display device (22) are used to achieve human-machine interaction, so as to send the control information to the implantable neurostimulator (1); the control information comprises an instruction for modifying the clinical stimulation parameters;
wherein the control information further comprises an up/down shift instruction, in response to which a main control chip (11) adjusts pulse intensity of the stimulation pulse sequence in a step-by-step manner.

2. The implantable nerve stimulator system according to claim 1, wherein the implantable neurostimulator (1) further comprises a post measurement feedback circuit (19) for measuring real-time stimulation parameters on the stimulation electrode (16), which are transmitted to the main control CPU (111) which stores the real-time stimulation parameters in the main control memory (112), and periodically sends the real-time stimulation parameters to the extracorporeal energy controller (2), or sends the real-time stimulation parameters to the extracorporeal energy controller (2) in response to a data read instruction; the extracorporeal energy controller (2) comprises a storage unit (23) to store the real-time stimulation parameters received from the implantable neurostimulator (1).

3. The implantable nerve stimulator system according to claim 1 or 2, wherein the extracorporeal energy controller (2) further comprises a storage unit (23) that stores operating program of the extracorporeal energy controller (2), information coming from the input device (20), and data received from the implantable neurostimulator (1);
the control information also comprises the data read instruction to read operation data comprising real-time stimulation parameters from the implantable neurostimulator (1) at any time.

4. The implantable nerve stimulator system according to claim 1, wherein the implantable nerve stimulator system further comprises an upper computer (3) serving as a control and information processing station, and the extracorporeal energy controller (2) further comprises an upper computer communication module (26), through which the upper computer (3) communicates with the extracorporeal energy controller (2), thereby sending an instruction to the implantable neurostimulator (1) through the extracorporeal energy controller (2), or reads data from the extracorporeal energy controller (2).

5. The implantable nerve stimulator system according to claim 4, wherein the upper computer communication module (26) is a wireless communication module such as Bluetooth.

6. The implantable nerve stimulator system according to claim 4, wherein the upper computer (3) has an upper computer software, through which a user sends an instruction to the extracorporeal energy controller (2) of a neural stimulation system to operate the extracorporeal energy controller of the neural stimulation system, or operate the implantable neurostimulator (1) through the extracorporeal energy controller of the neural stimulation system, the operations comprising setting, measuring, programming and data management of the clinical stimulation parameters, the data involved in the data management comprising measurable relevant parameters, modified data and observation variables, and the relevant parameters being generated during operation of the extracorporeal energy controller (2) and the implantable neurostimulator (1) of the neural stimulation system.

7. The implantable nerve stimulator system according to claim 6, wherein the upper computer software is capable of operating the upper computer (3) to connect to a network or an internal server for backup and update of the program and data.

## Patentansprüche

1. Implantierbares Nervenstimulationssystem, umfassend einen implantierbaren Neurostimulator (1), aufweisend ein Stimulationsantennenmodul und eine extrakorporale Energiesteuerung (2), aufweisend ein Energiesteuerungsantennenmodul (21), wobei, durch das Stimulationsantennenmodul und das Energiesteuerungsantennenmodul (21), der implantierbare Neurostimulator (1) mit der extrakorporalen Energiesteuerung (2) kommuniziert und elektrische Energie von dieser durch Hochfrequenz empfängt, wobei der implantierbare Neurostimulator (1) eine Hauptsteuerungs-CPU (111), einen Hauptsteuerungsspeicher (112) zum Speichern von Steuerinformationen mit klinischen Stimulationsparametern und Stimulationselektroden (16) aufweist und die Hauptsteuerungs-CPU (111) die klinischen Stimulationsparameter verwendet, um Stimulationspulssequenzen aktiv zu generieren, und die Stimulationspulssequenzen auf die Stimulationselektroden (16) anwendet;
wobei der implantierbare Neurostimulator (1) ferner eine Gleichrichtungsenergiespeicherschaltung (13) zum Speichern der empfangenen elektrischen Energie und eine Frontmessungsrückkopplungsschaltung (10) zum Messen der Speichermenge der elektrischen Energie in der Gleichrichtungsenergiespeicherschaltung (13) umfasst, und, wenn die Speichermenge der elektrischen Energie nicht ausreicht, die Hauptsteuerungs-CPU (111) eine Leistungsanpassungsanweisung an die extrakorporale Energiesteuerung (2) sendet, welche die Übertragungsleistung basierend auf der empfangenen Leistungsanpassungsanweisung anpasst;
wobei die extrakorporale Energiesteuerung (2) ferner eine Eingabevorrichtung (20), eine Anzeigevorrichtung (22) und eine Stromversorgung (24) umfasst; die Eingabevorrichtung (20) und die Anzeigevorrichtung (22) verwendet werden, um eine Mensch-Maschine-Interaktion zu erzielen, um so die Steuerinformationen an den implantierbaren Neurostimulator zu senden (1); die Steuerinformationen eine Anweisung zum Modifizieren der klinischen Stimulationsparameter umfasst;
wobei die Steuerinformationen ferner eine Auf-/Abwärtsverschiebungsanweisung umfassen, in Reaktion auf welche ein Hauptsteuerchip (11) eine Pulsintensität der Stimulationspulssequenz schrittweise anpasst.

2. Implantierbares Nervenstimulationssystem gemäß Anspruch 1, wobei der implantierbare Neurostimulator (1) ferner eine Rückkopplungsschaltung nach Messungen (19) zum Messen von Echtzeit-Stimulationsparametern an der Stimulationselektrode (16) umfasst, die an die Hauptsteuerungs-CPU (111) übertragen werden, welche die Echtzeit-Stimulationsparameter im Hauptsteuerungsspeicher (112) speichert und die Echtzeit-Stimulationsparameter periodisch an die extrakorporale Energiesteuerung (2) sendet oder die Echtzeit-Stimulationsparameter in Reaktion auf eine Datenleseanweisung an die extrakorporale Energiesteuerung (2) sendet; wobei die extrakorporale Energiesteuerung (2) eine Datenspeichereinheit (23) zum Speichern der Echtzeit-Stimulationsparameter umfasst, die von dem implantierbaren Neurostimulator (1) empfangen werden.

3. Implantierbares Nervenstimulationssystem gemäß Anspruch 1 oder 2, wobei die extrakorporale Energiesteuerung (2) ferner eine Datenspeichereinheit (23) umfasst, die ein Betriebsprogramm der extrakorporalen Energiesteuerung (2), von der Eingabevorrichtung (20) eingehende Informationen und von dem implantierbaren Neurostimulator (1) empfangene Daten speichert;
wobei die Steuerinformationen außerdem die Datenleseanweisung zum Auslesen von Betriebsdaten umfassen, umfassend Echtzeit-Stimulationsparameter von dem implantierbaren Neurostimulator (1) zu jedem Zeitpunkt.

4. Implantierbares Nervenstimulationssystem gemäß Anspruch 1, wobei das implantierbare Nervenstimulationssystem ferner einen oberen Computer (3) umfasst, der als Steuer- und Informationsverarbeitungsstation fungiert, und die extrakorporale Energiesteuerung (2) ferner ein oberes Computerkommunikationsmodul (26) umfasst, durch welches der obere Computer (3) mit der extrakorporalen Energiesteuerung (2) kommuniziert, wodurch eine Anweisung an den implantierbaren Neurostimulator (1) durch die extrakorporale Energiesteuerung (2) gesendet wird, oder Daten von der extrakorporalen Energiesteuerung (2) ausliest.

5. Implantierbares Nervenstimulationssystem gemäß Anspruch 4, wobei das obere Computerkommunikationsmodul (26) ein drahtloses Kommunikationsmodul wie etwa Bluetooth ist.

6. Implantierbares Nervenstimulationssystem gemäß Anspruch 4, wobei der obere Computer (3) eine obere Computersoftware aufweist, durch welche ein Benutzer eine Anweisung an die extrakorporale Energiesteuerung (2) eines Nervenstimulationssystems sendet, um die extrakorporale Energiesteuerung des Nervenstimulationssystems zu betreiben oder den implantierbaren Neurostimulator (1) durch die extrakorporale Energiesteuerung des Nervenstimulationssystems zu betreiben, wobei der Betrieb das Einstellen, Messen, Programmieren und die Datenverwaltung der klinischen Stimulationsparameter umfasst, die in die Datenverwaltung einbezogenen Daten messbare relevante Parameter, modifizierte Daten und Beobachtungsvariablen umfassen und die relevanten Parameter während des Betriebs der extrakorporalen Energiesteuerung (2) und des implantierbaren Neurostimulators (1) des Nervenstimulationssystems generiert werden.

7. Implantierbares Nervenstimulationssystem gemäß Anspruch 6, wobei die obere Computersoftware in der Lage ist, den oberen Computer (3) zu betreiben, um eine Verbindung zu einem Netzwerk oder einem internen Server für Sicherungen und Aktualisierungen des Programms und der Daten herzustellen.

## Revendications

1. Système de stimulateur nerveux implantable, comprenant un neurostimulateur implantable (1) ayant un module d'antenne stimulateur et un dispositif de commande d'énergie extracorporelle (2) ayant un module d'antenne de dispositif de commande d'énergie (21), au moyen du module d'antenne stimulateur et du module d'antenne de dispositif de commande d'énergie (21), le neurostimulateur implantable (1) communiquant avec le dispositif de commande d'énergie extracorporelle (2) et recevant de l'énergie électrique de celui-ci par radiofréquence, le neurostimulateur implantable (1) ayant une unité centrale de commande principale (111), une mémoire de commande principale (112) pour stocker des informations de commande contenant des paramètres de stimulation clinique, et des électrodes de stimulation (16), et l'unité centrale de commande principale (111) utilisant les paramètres de stimulation clinique pour générer activement des séquences d'impulsions de stimulation et appliquant les séquences d'impulsions de stimulation aux électrodes de stimulation (16) ;
le neurostimulateur implantable (1) comprenant en outre un circuit de stockage d'énergie de rectification (13) pour stocker l'énergie électrique reçue et un circuit de rétroaction de mesure frontale (10) pour mesurer la quantité de stockage d'énergie électrique dans le circuit de stockage d'énergie de rectification (13), et lorsque la quantité de stockage d'énergie électrique est insuffisante, l'unité centrale de commande principale (111) envoyant une instruction de réglage de puissance au dispositif de commande d'énergie extracorporelle (2) qui ajuste la puissance de transmission sur la base de l' instruction de réglage de puissance ;
le dispositif de commande d'énergie extracorporelle (2) comprenant en outre un dispositif d'entrée (20), un dispositif d'affichage (22) et une alimentation électrique (24) ; le dispositif d'entrée (20) et le dispositif d'affichage (22) étant utilisés pour réaliser une interaction homme-machine, de manière à envoyer les informations de commande au neurostimulateur implantable (1) ; les informations de commande comprenant une instruction pour modifier les paramètres de stimulation clinique ;
les informations de commande comprenant en outre une instruction de passage au rapport supérieur/inférieur, en réponse à laquelle une puce de commande principale (11) ajuste l'intensité des impulsions de la séquence d'impulsions de stimulation de manière progressive.

2. Système de stimulateur nerveux implantable selon la revendication 1, le neurostimulateur implantable (1) comprenant en outre un circuit de rétroaction post-mesure (19) pour mesurer des paramètres de stimulation en temps réel sur l'électrode de stimulation (16), qui sont transmis à l'unité centrale de commande principale (111) qui stocke les paramètres de stimulation en temps réel dans la mémoire de commande principale (112), et envoie périodiquement les paramètres de stimulation en temps réel au dispositif de commande d'énergie extracorporelle (2), ou envoie les paramètres de stimulation en temps réel au dispositif de commande d'énergie extracorporelle (2) en réponse à une instruction de lecture de données ; le dispositif de commande d'énergie extracorporelle (2) comprenant une unité de stockage (23) pour stocker les paramètres de stimulation en temps réel reçus du neurostimulateur implantable (1).

3. Système de stimulateur nerveux implantable selon la revendication 1 ou 2, le dispositif de commande d'énergie extracorporelle (2) comprenant en outre une unité de stockage (23) qui stocke le programme de fonctionnement du dispositif de commande d'énergie extracorporelle (2), des informations provenant du dispositif d'entrée (20), et des données reçues du neurostimulateur implantable (1) ;
les informations de commande comprenant également l'instruction de lecture de données pour lire des données d'opération comprenant des paramètres de stimulation en temps réel provenant du neurostimulateur implantable (1) à tout moment.

4. Système de stimulateur nerveux implantable selon la revendication 1, le système de stimulateur nerveux implantable comprenant en outre un ordinateur supérieur (3) servant de station de commande et de traitement d'informations, et le dispositif de commande d'énergie extracorporelle (2) comprenant en outre un module de communication d'ordinateur supérieur (26), au moyen duquel l'ordinateur supérieur (3) communique avec le dispositif de commande d'énergie extracorporelle (2), envoyant ainsi une instruction au neurostimulateur implantable (1) au moyen du dispositif de commande d'énergie extracorporelle (2), ou lisant des données du dispositif de commande d'énergie extracorporelle (2).

5. Système de stimulateur nerveux implantable selon la revendication 4, le module de communication d'ordinateur supérieur (26) étant un module de communication sans fil tel que Bluetooth.

6. Système de stimulateur nerveux implantable selon la revendication 4, l'ordinateur supérieur (3) ayant un logiciel d'ordinateur supérieur, au moyen duquel un utilisateur envoie une instruction au dispositif de commande d'énergie extracorporelle (2) d'un système de stimulation neuronale pour faire fonctionner le dispositif de commande d'énergie extracorporelle du système de stimulation neuronale, ou pour faire fonctionner le neurostimulateur implantable (1) au moyen du dispositif de commande d'énergie extracorporelle du système de stimulation neuronale, les opérations comprenant le réglage, la mesure, la programmation et la gestion des données des paramètres de stimulation clinique, les données impliquées dans la gestion des données comprenant des paramètres pertinents mesurables, des données modifiées et des variables d'observation, et les paramètres pertinents étant générés pendant le fonctionnement du dispositif de commande d'énergie extracorporelle (2) et du neurostimulateur implantable (1) du système de stimulation neuronale.

7. Système de stimulateur nerveux implantable selon la revendication 6, le logiciel d'ordinateur supérieur étant capable de faire fonctionner l'ordinateur supérieur (3) pour se connecter à un réseau ou à un serveur interne pour sauvegarder et mettre à jour le programme et les données.
